(19) Europäisches Patentamt
European Patent Office
Office européen des brevets

(11) Publication number: **0 118 120**
**B1**

(12) **EUROPEAN PATENT SPECIFICATION**

(45) Date of publication of patent specification: **14.06.89**

(21) Application number: **84102235.3**

(22) Date of filing: **02.03.84**

(51) Int. Cl.[4]: **C 07 D 405/04,** C 07 D 409/04 // A61K31/445, C07D319/12, C07D339/08

(54) **1,4-Dihydropyridine compound.**

(30) Priority: **04.03.83 JP 34639/83**
**23.08.83 JP 152405/83**

(43) Date of publication of application:
**12.09.84 Bulletin 84/37**

(45) Publication of the grant of the patent:
**14.06.89 Bulletin 89/24**

(84) Designated Contracting States:
**DE FR GB IT**

(56) References cited:
**EP-A-0 087 156**

(73) Proprietor: **NIKKEN CHEMICALS CO., LTD.**
**4-14, Tsukiji 5-chome Chuo-ku**
**Tokyo (JP)**

(73) Proprietor: **INSTITUTE OF ORGANIC SYNTHESIS ACADEMY OF SCIENCES LATVIAN SSR.**
**Aizkraukles 21**
**Riga-6 (SU)**

(72) Inventor: **Suzuki, Kenichi**
**12-12, Miyashirodai 2-chome Miyashiromachi**
**Minamisaitama-gun Saitama-ken (JP)**
Inventor: **Shiraishi, Takanori**
**9-13, Ohara 7-chome**
**Urawa-shi Saitama-ken (JP)**
Inventor: **Yoshida, Yohji**
**14-11, Seiji 2-chome Sugitomachi**
**Kitakatsushika-gun Saitama-ken (JP)**
Inventor: **Matsumoto, Toshio**
**747-6, Koshikiya**
**Ageo-shi Saitama-ken (JP)**
Inventor: **Iwamoto, Mitsuo**
**8-1-532, Oaza Niizutsumi**
**Omiya-shi Saitama-ken (JP)**
Inventor: **Motoyoshi, Yoshie**
**17-3, Sugamo 1-chome**
**Toshima-ku Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 118 120 B1**

Courier Press, Leamington Spa, England.

(72) Inventor: Niwa, Hirosuke
16-8, Asahi 6-chome
Kawaguchi-shi Saitama-ken (JP)
Inventor: Inoue, Yoichi
169-7, Horisaki
Omiya-shi Saitama-ken (JP)

(74) Representative: Kador . Klunker . Schmitt-Nilson . Hirsch
Corneliusstrasse 15
D-8000 München 5 (DE)

**Description**

The present invention relates to a novel 1,4-dihydropyridine compound having the general formula:

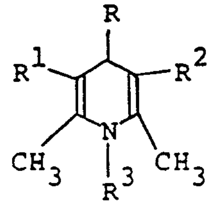

(I)

wherein R is 2-(5,6-dihydro-p-dioxinyl)

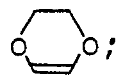

$R^1$ and $R^2$ are independently a cyano group —CN, an acetyl group —$COCH_3$, or a substituted oxycarbonyl group having the general formula $COOR^4$, wherein $R^4$ is a methyl —$CH_3$, isopropyl —$CH(CH_3)_2$, allyl —$CH_2$—$CH{=}CH_2$, propargyl —$CH_2$—$C{\equiv}CH$, methoxymethyl —$CH_2OCH_3$, 2-methoxyethyl —$CH_2CH_2OCH_3$, or 1-methyl-2-methoxyethyl —$CH(CH_3)CH_2OCH_3$ group, provided that both $R^1$ and $R^2$ are not —$COOCH_3$; and $R^3$ is hydrogen.

The 1,4-dihydropyridine compounds having the above-mentioned general formula [I] are novel compounds which have not been reported in any literature. These compounds have a remarkable liver protective action and a low toxicity, as mentioned hereinbelow. Accordingly, these compounds are useful as medicine for the treatment of hepatic disease. Especially, the 1,4-dihydropyridine compounds having the general formula (I) in which $R^1$ and $R^2$ are independently an acetyl, methyloxycarbonyl, methoxymethyloxycarbonyl, or 2-methoxyethyloxycarbonyl group are those having an especially remarkable liver protective action. Examples of these preferable 1,4-dihydropyridine compounds are 3,5-diacetyl-4-[2-(5,6-dihydro-p-dioxinyl)]-2,6-dimethyl-1,4-dihydropyridine, bis(2-methioxyethyl) 4-[2-(5,6-dihydro-p-dioxinyl)]-2,6-dimethyl-1,4-dihydropyridine-3,5-dicarboxylate, methyl 5-acetyl-4-[2-(5,6-dihydro-p-dioxinyl)]-2,6-dimethyl-1,4-dihydropyridine-3-carboxylate, bis(methoxymethyl) 4-[2-(5,6-dihydro-p-dioxinyl)]-2,6-dimethyl-1,4-dihydropyridine-3,5-dicarboxylate.

The 1,4-dihydropyridine compounds of the present invention having the general formula:

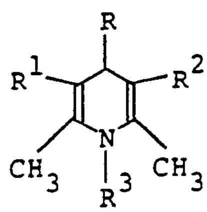

(I')

wherein R and $R^3$ are the same as defined above, and $R^1$ and $R^2$ are independently a cyano group, an acetyl group, or a substituted oxycarbonyl group having the general formula $COOR^4$ wherein $R^4$ is a methyl, isopropyl, allyl, propargyl, 2-methoxyethyl, or 1-methyl-2-methoxy-ethyl group provided that both $R^1$ and $R^2$ are not —$COOCH_3$, can be prepared by reacting (i) an aldehyde having the general formula:

$$R\text{—}CHO \qquad \text{(II)}$$

wherein R is the same as defined above, (ii) a compound having the general formula:

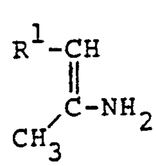

(III)

wherein $R^1$ is the same as defined in the formula (I'), and (iii) a compound having the general formula:

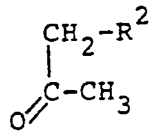

(IV)

wherein $R^2$ is the same as defined in the formula (I') together in the presence, or in the absence, of an organic solvent upon heating at a temperature of 60°C to 170°C, preferably under a reflux condition.

Furthermore, the 1,4-dihydropyridine compounds of the present invention having the general formula:

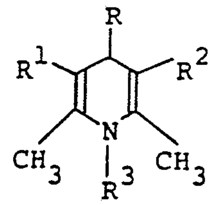

(I")

wherein R and $R^3$ are the same as defined above, and $R^1$ and $R^2$ are a methoxymethyl oxycarbonyl group ($-COOCH_2OCH_3$) can be prepared by reacting (iv) an aldehyde having the above-mentioned general formula (II), (v) a compound having the general formula:

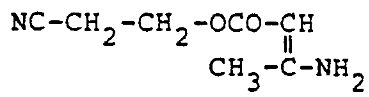

(V)

and (vi) a compound having the general formula:

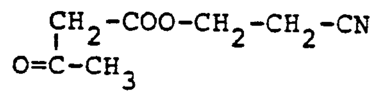

(VI)

together in the presence, or in the absence, of an organic solvent to form a compound having the general formula:

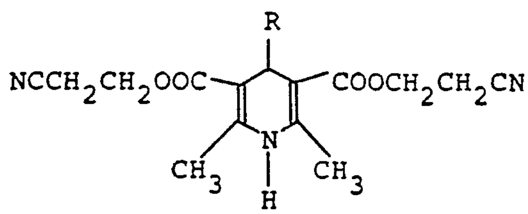

(VII)

wherein R is the same as defined above, and then hydrolyzing the compound (VII). The resultant hydrolyzate of the compound (VII) is reacted with an approximate stoichiometric amount of chloromethyl methyl ether to form the 1,4-dihydropyridine compounds of the general formula (I'').

The preparation method of the 1,4-dihydropyridine compound having the general formula (I) is further explained in detail hereinbelow.

(a) In the case of both $R^1$ and $R^2$ in the general formula (I) being a cyano group, the desired compound can be prepared by reacting 2-formyl-p-dioxene with and β-aminocrotononitrile in, for example, acetic acid, as follows:

Method 1

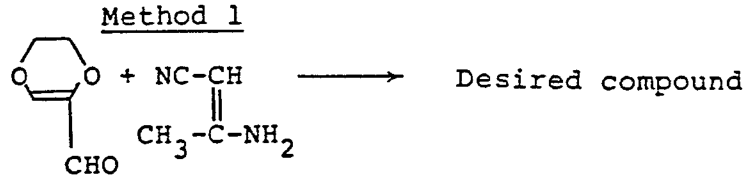


(b) In the case of $R^1$ being a cyano group and $R^2$ being an acetyl group in the general formula (I) the desired compound can be prepared by reacting 2-formyl-p-dioxene, β-aminocrotononitrile, and acetylaceton in the presence, or in the absence, of an organic solvent such as ethanol, n-propanol, isopropanol, or pyridine upon heating at a temperature of 60°C to 170°C, preferably under a reflux condition as follows:

Method 2

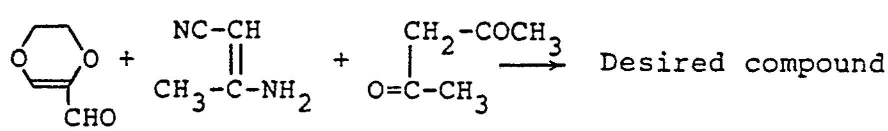


(c) In the case of $R^1$ being a cyano group and $R^2$ being a substituted oxycarbonyl group in the general formula (I), the desired compound can be prepared by reacting 2-formyl-p-dioxene, β-aminocrotononitrile,

and acetoacetic ester in the presence, or in the absence, of an organic solvent as used in method 2 upon heating at a temperature of 60°C to 170°C, preferably under a reflux condition as follows:

Method 3

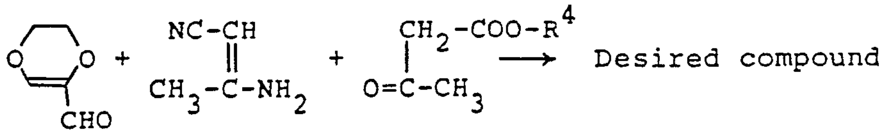


(d) In the case of both $R^1$ and $R^2$ being acetyl groups in the general formula (I'), the desired compound can be prepared by reacting 2-formyl-p-dioxene, 4-amino-3-penten-2-one, and acetyl acetone in the presence, or in the absence, of an organic solvent as mentioned above upon heating at a temperature of 60°C to 170°C or under a reflux condition as follows:

Method 4

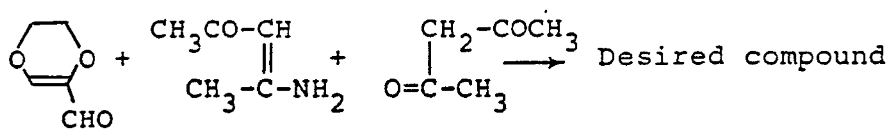


(e) In the case of $R^1$ being an acetyl group and $R^2$ being a substituted oxycarbonyl group in the general formula (I'), the desired compound can be prepared by reacting 2-formyl-p-dioxene, 4-amino-3-penten-2-one, and acetoacetic ester in the presence, or in the absence, of the above-mentioned organic solvent upon heating at a temperature of 60°C to 170°C or under a reflux condition as shown in the following method 5, or by first reacting 2-formyl-p-dioxene and acetoacetic ester to form 1-[2-(5,6-dihydro-p-dioxinyl)]-3-oxo-1-butene-2-carboxylic ester, which is in turn reacted with 4-amino-3-penten-2-one in the presence, or in the absence, of the above-mentioned organic solvent upon heating at a temperature of 60°C to 170°C or under a reflux condition as shown in the following method 6.

Method 5

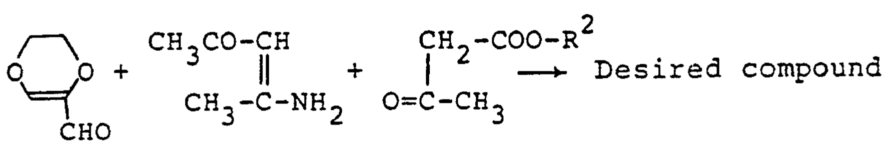


Method 6

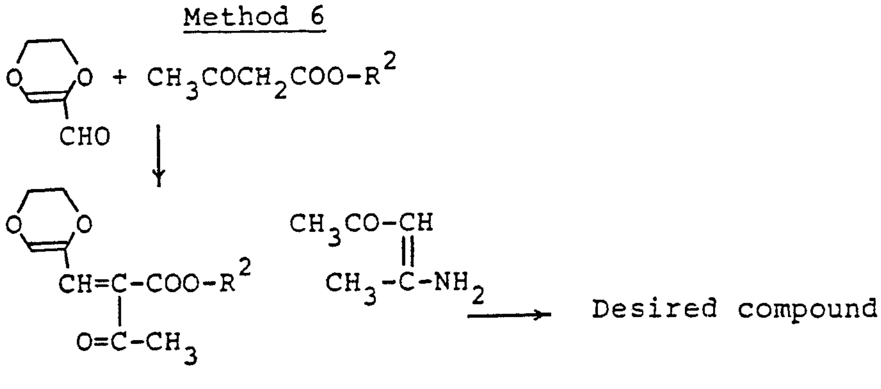


(f) In the case of both $R^1$ and $R^2$ being substituted oxycarbonyl groups except for a methoxymethyl oxycarbonyl group in the above-mentioned general formula (I'), the desired compound can be prepared by reacting 2-formyl-p-dioxene, β-amino crotonic ester, and acetoacetic ester in the presence, or in the absence, of the above-mentioned organic solvent upon heating at a temperature of 60°C to 170°C or under a reflux condition as shown in the following method 7, or by reacting 1-[2-(5,6-dihydro-p-dioxinyl)]-3-oxo-1-butene-2-carboxylic acid ester obtained in the same manner as in the method 6 with β-aminocrotonic ester in the presence, or in the absence, of the above-mentioned organic solvent upon heating at a temperature of 60°C to 170°C or under a reflux condition as shown in the following method 8, or by reacting 2-formyl-p-dioxene, acetoacetic ester, and aqueous ammonia in the presence, or in the absence, of the above-mentioned organic solvent upon heating at a temperature of 60°C to 170°C or under a reflux condition as shown in the following method 9.

Method 7

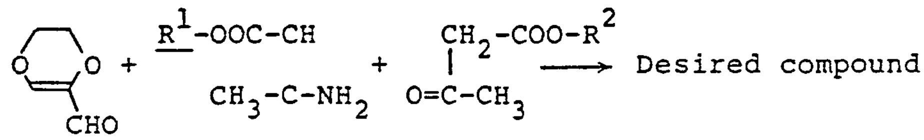


Method 8

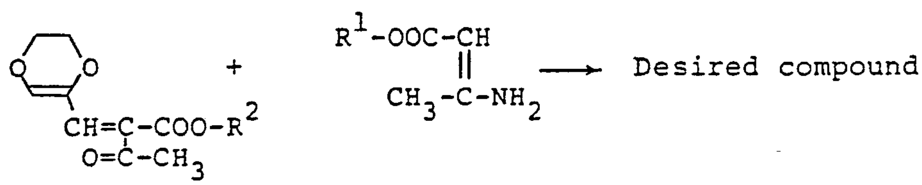


Method 9

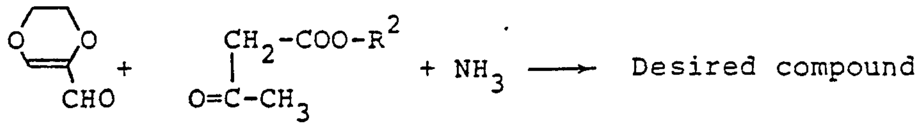


(g) In the case of $R^1$ and/or $R^2$ being a methoxymethyl oxycarbonyl group or groups in the general formula (I''), the 1,4-dihydropyridine compound having the general formula (I') in which $R^1$ and/or $R^2$ are 2-cyanoethyl oxycarbonyl groups is first prepared by any one of the above-mentioned methods 3 and 5 to 9 and, then, the resultant compound (X) is hydrolyzed by, for example, sodium hydroxide. The resultant hydrolysate (XI) is reacted with chloromethyl methyl ether in, for example, an organic solvent such as dimethyl formamide to form the desired compound (XII).

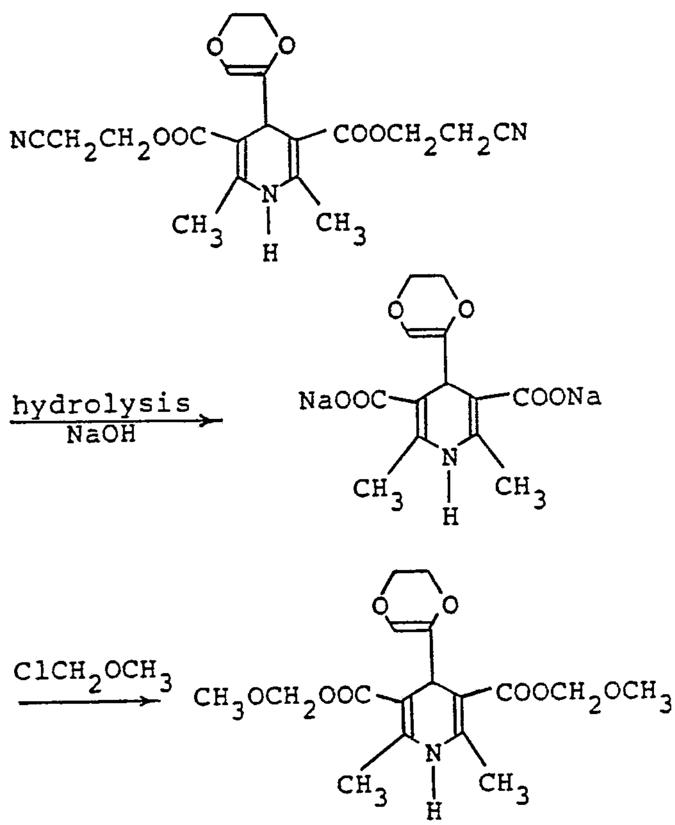


(X)

(XI)

(XII)

The above-mentioned 1,4-dihydropyridine compounds [I] can also be prepared by the known reactions set forth in, for example, Japanese Examined Patent Publication (Kokoku) Nos. 46—40625 and 56—37225 and Japanese Unexamined Patent Publication (Kokai) Nos. 51—108075, 53—92784, 54—103876, 55—64570, 55—89281, and 58—159490. (In the last document the disclaimed compounds are described, however the document is not relevant to the question of inventive step).

The above-mentioned starting compounds used in the above-mentioned reactions are almost of known compounds which are readily purchased, or can be readily prepared by those skilled in the art. For instance, β-aminocrotono nitrile, acetoacetic esters, β-aminocrotonic esters, acetylacetone, and 4-amino-3-penten-2-one are those which are conventionally used as a starting compound for the production of 1,4-dihydropyridine compounds and which are commercially available on the market. The 2-formyl-p-dioxene among the aldehydes shown in formula (II) can be prepared from p-dioxene via 2-ethoxy-3-diethoxymethyl-p-dioxane according to a method described in, for example, M. S. Shostakovskii, Izvest. Akad. Nauk. S.S.S.R. Otdel, Khim, Nauk., 1685, (1961).

The 1,4-dihydropyridine compounds [I] formed in the above-mentioned reactions can be separated from the reaction mixtures and can be purified in any conventional techniques including solvent extraction, chromatographic separation, crystallization from an appropriate solvent.

Although there is no limitation in the mole ratio of the starting compounds, these compounds can be desirably used in an approximate equimolar amount.

As is clear from the results shown in Examples below, the present compounds are effective for the prevention and treatment of hepatic disorders and also have a low toxicity. Accordingly, it is clear that the present compounds are a medicine for treatment of hepatic disease caused by various factors.

The present invention will be further illustrated by, but is by no means limited to, the following examples illustrating the synthesis of the present compounds [I] as well as the pharmacological test data for the evaluation thereof.

Example 1 (Synthesis Example)

Synthesis of 3,5-dicyano-4-[2-(5,6-dihydro-p-dioxinyl)]-2,6-dimethyl-1,4-dihydropyridine

A 7.72 g (0.08 mol) amount of β-aminocrotononitrile having a purity of 85% was dissolved in 20 ml of acetic acid. 4.56 g (0.04 mol) of 2-formyl-p-dioxene was dissolved in 8 ml of acetic acid. The 2-formyl-p-dioxene solution was dropwise added to the β-aminocrotonitrile solution with stirring, while the temperature was maintained at 20°C or less. Thereafter, the temperature of the reaction mixture was raised to 60°C and the reaction mixture was stirred for 5 minutes. After cooling, the reaction mixture was concentrated until dryness. The resultant residue was dissolved in ethyl acetate and the solution was washed with an aqueous sodium bicarbonate solution and water. The solution was dried over anhydrous sodium sulfate and was evaporated to remove the solvent.

The resultant residue was subjected to chromatographic purification by using a column packed with 160 g of silica gel. A mixed solvent of chloroform and ethyl acetate (3:1) (V/V) was used as an eluent. The compound thus purified was recrystallized twice from ethanol to give 2.06 g (21.2% yield) of the desired product in the form of white crystals.

The analytical data of this product were as follows:

m.p.: 212.0—213.5°C

IR$\nu_{max}^{KBr}$cm$^{-1}$: 3400 (NH), 3260 (NH), 2210 (CN), 1660 (C=C)

NMR (DMSO-$d_6$, DSS)δ: 2.01 (6H, s, 2,6—$CH_3$), 3.70 (1H, s, 4—H), 4.01 (4H, s, O—$CH_2$—$CH_2$—O), 6.09 (1H, s, vinyl-H), 9.30 (1H, br, NH)

Elementary analysis data (for $C_{14}H_{13}N_3O_2$)

Calculated: C, 64.19; H, 5.39; N, 17.27(%)

Found: C, 63.92; H, 5.46; N, 17.01(%)

Example 2 (Synthesis Example)

Synthesis of 3-acetyl-5-cyano-4-[2-(5,6-dihydro-p-dioxinyl)]-2,6-dimethyl-1,4-dihydropyridine

A 1.93 g (0.02 mol) amount of β-aminocrotononitrile having a purity of 85%, 1.93 g (0.02 mol) of acetylacetone, and 2.28 g (0.02 mol) of 2-formyl-p-dioxene were dissolved in 10 ml of isopropanol and the resultant solution was heated under reflux for 3 hours. After cooling, the reaction mixture was evaporated until dryness.

The resultant residue was subjected to chromatographic purification by using a column packed with 300 g of silica gel. A mixed solvent of chloroform and ethyl acetate (3:1) (V/V) was used as an eluent. The compound thus purified was crystallised from a mixed solvent of diethyl ether and diisopropyl ether. The

crystal thus obtained was recrystallized from ethanol to give 0.82 g of the desired product in the form of pale yellow crystals.

The analytical data of this product were as follows:

m.p.: 172.0—174.0°C

$IR\nu_{max}^{KBr}cm^{-1}$: 3280 (NH), 2260 (CN), 1680 (C=C)

NMR (DMSO-$d_6$, DSS)δ: 2.06 (3H, s, 6—$CH_3$), 2.20 (6H, s, 2—$CH_3$, CO—$CH_3$), 3.97 (5H, s, 4—H, O—$CH_2$—$CH_2$—O), 5.94 (1H, s, vinyl-H), 9.20 (1H, br, NH)

Elementary analysis data (for $C_{14}H_{16}N_2O_3$)

Calculated: C, 64.60; H, 6.20; N, 10.76(%)

Found: C, 64.44; H, 6.19; N, 10.82(%)

Example 3 (Synthesis Example)

Synthesis of methyl 5-cyano-4-[2-(5,6-dihydro-p-dioxinyl)]-2,6-dimethyl-1,4-dihydropyridine-3-carboxylate

A 5.79 g (0.06 mol) amount of β-aminocrotononitrile having a purity of 85%, 6.96 g (0.06 mol) of methyl acetoacetate, and 6.84 g (0.06 mol) of 2-formyl-p-dioxene were dissolved in 30 ml of isopropanol and the solution thus obtained was heated under reflux for 16 hours. After cooling, the reaction solution was evaporated until dryness.

The resultant residue was subjected to chromatographic purification by using a column packed with 280 g of silica gel. A mixed solvent of chloroform and methanol (80:1) (V/V) was used as an eluent. The compound thus purified was recrystallized from ethyl acetate to give 1.04 g of the desired product in the form of pale yellow crystals.

The analytical data of this product were as follows:

m.p.: 185.5—187.0°C

$IR\nu_{max}^{KBr}cm^{-1}$: 3300 (NH), 2200 (CN), 1700 (C=O), 1250 (C—O)

NMR (DMSO-$d_6$, DSS)δ: 2.06 (3H, s, 6—$CH_3$), 2.23 (3H, s, 2—$CH_3$), 3.63 (3H, s, O—$CH_3$), 3.87 (1H, s, 4—H), 3.96 (4H, s, O—$CH_2$—$CH_2$—O), 5.88 (1H, s, vinyl-H), 9.25 (1H, br, NH)

Elementary analysis data (for $C_{14}H_{16}N_2O_4$)

Calculated: C, 60.86; H, 5.84; N, 10.14(%)

Found: C, 60.65; H, 5.82; N, 10.11(%)

Example 4 (Synthesis Example)

Synthesis of bis(1-methyl-2-methoxyethyl) 4-[2-(5,6-dihydro-p-dioxinyl)]-2,6-dimethyl-1,4-dihydropyridine-3,5-dicarboxylate

A 4.70 g (0.027 mol) amount of 1-methyl-2-methoxyethyl acetoacetate, 4.68 g (0.027 mol) of 1-methyl-2-methoxyethyl β-amino crotonate, and 3.08 g (0.027 mol) of 2-formyl-p-dioxene were mixed and the mixture was then heated at a temperature of 150°C for 16 hours while stirring. After cooling, the reaction mixture was subjected to chromatographic purification by using a column packed with 320 g of silica gel. A mixed solvent of benzene and acetone (5:1) (V/V) was used as an eluent. The product thus purified was crystallized from a mixed solvent of diethyl ether and diisopropyl ether, and the crystal thus obtained was recrystallized from a mixed solvent of diethyl ether, diisopropyl ether, and ethanol. As a result, 0.56 g of the desired product in the form of pale yellow crystals was obtained.

The analytical data of the desired product were as follows:

m.p.: 120.0—122.0°C

$IR\nu_{max}^{KBr}cm^{-1}$: 3250 (NH), 1700 (C=O), 1270 (C—O)

NMR ($CDCL_3$, TMS)δ: 1.25 (6H, d, 2 × CH—$CH_3$), 2.27 (6H, s, 2,6—$CH_3$), 3.36 (6H, s, 2 × O—$CH_3$), 3.45 (4H, d, 2 × CH—$CH_2$—O), 3.91 (4H, s, O—$CH_2$—$CH_2$—O), 4.44 (1H, s, 4—H), 5.08 (2H, m, 2 × COO—CH), 5.85 (1H, s, vinyl-H), 6.16 (1H, br, NH)

Elementary analysis data (for $C_{21}H_{31}NO_8$)

Calculated: C, 59.28; H, 7.34; N, 3.29(%)

Found: C, 59,26; H, 7.16; N, 3.37(%)

Example 5 (Synthesis Example)

Synthesis of bis(2-cyanoethyl) 4-[2-(5,6-dihydro-p-dioxinyl)] 2,6-dimethyl-1,4-dihydropyridine-3,5-dicarboxylate

A 2.30 g (0.02 mol) amount of 2-formyl-p-dioxene, 5.80 g (0.037 mol) of 2-cyanoethyl acetoacetate, and 3.5 ml of 28% aqueous ammonia were dissolved in 30 ml of isopropanol. The resultant solution was heated under reflux for 24 hours. After cooling, the reaction solution was concentrated and the precipitated crystal

was separated by filtration. The crystal was recrystallized from ethanol to give 2.76 g (35.3% yield) of the desired product in the form of pale yellow crystals.

The analytical data of the product were as follows:

m.p.: 135.5—137.0°C

$IR\nu_{max}^{KBr}cm^{-1}$: 3345 (NH), 2325 (CN), 1695 (C=O), 1200 (C—O), 1120 (C—O)

NMR ($CD_3OD$, TMS)δ: 2.31 (6H, s, 2,6—$CH_3$), 2.75 (4H, t, 2 × COO—$CH_2$—$CH_2$—CN), 3.93 (4H, s, O—$CH_2$—$CH_2$—O), 4.34 (4H, t, 2 × COO—$CH_2$—$CH_2$—CN), 4.45 (1H, s, 4—H), 5.91 (1H, s, vinyl-H), 6.32 (1H, br, NH)

Elementary analysis data (for $C_{19}H_{21}N_3O_6$)

Calculated: C, 58.91; H, 5.46; N, 10.85(%)

Found: C, 58.67; H, 5.55; N, 10.58(%)

Example 6 (Synthesis Example)

Synthesis of bis(methoxymethyl) 4-[2-(5,6-dihydro-p-dioxinyl)]-2,6-dimethyl-1,4-dihydropyridine-3,5-dicarboxylate

A 3.30 g (8.52 mmol) amount of bis(2-cyanoethyl) 4-[2-(5,6-dihydro-p-dioxinyl)]-2,6-dimethyl-1,4-dihydropyridine-3,5-dicarboxylate was dissolved in 10 ml of dimethoxyethane and then, to this solution, 50 ml of water and 17.1 ml of a 1N sodium hydroxide solution were added. The mixture was stirred at a room temperature for 1 hour and the solvent was then distilled in vacuo on a water bath at a temperature of 30°C. To the resultant residue, anhydrous dimethyl formamide was added and suspended with stirring. 2.0 g (25 mmol) of chloromethyl methyl ether was added thereto and the mixture was allowed to stand with stirring overnight at a room temperature.

Thereafter, the solvent was concentrated until dryness and the residue was then dissolved in ethyl acetate. The solution was washed with water and dried over anhydrous sodium sulfate. The solvent was then distilled off. The resultant residue was subjected to chromatographic purification by using a column packed with 50 g of silica gel. A mixture of n-hexane and ethyl acetate (1:1) (V/V) was used as an eluent. The resultant crystal was recrystallized from ethanol to give 0.81 g (25.7% yield) of the desired product in the form of a white crystal.

The analytical data of the product were as follows:

m.p.: 105.5—106.5°C

$IR\nu_{max}^{KBr}cm^{-1}$: 3345 (NH), 1695 (C=O), 1660 (C=C), 1200 (C—O), 1075 (C—O)

NMR ($CDCl_3$, TMS)δ: 2.33 (6H, s, 2,6—$CH_3$), 3.48 (6H, s, 2 × O—$CH_3$), 3.93 (4H, s, O—$CH_2$—$CH_2$—O), 4.53 (1H, s, 4—H), 5.23, 5.39 (4H, ABq, J=6.6 Hz, 2 × O—$CH_2$—O—$CH_3$), 5.93 (1H, s, vinyl-H), 6.15 (1H, br, NH)

Elementary analysis data (for $C_{17}H_{23}NO_8$)

Calculated: C, 55.28; H, 6.28; N, 3.79(%)

Found: C, 55.44; H, 6.24; N, 3.90(%)

Example 7 (Synthesis Example)

Synthesis of diisopropyl 4-[2-(5,6-dihydro-p-dioxinyl)]-2,6-dimethyl-1,4-dihydropyridine-3,5-dicarboxylate

To a solution of 7.21 g (0.05 mol) of isopropyl acetoacetate in 20 ml of isopropanol, gaseous ammonia was bubbled for one hour and the solution was allowed to stand overnight at a room temperature. The solvent was then distilled off.

Thereafter, to the resultant residue, 7.21 g (0.05 mol) of isopropyl acetoacetate, 5.71 g (0.05 mol) of 2-formyl-p-dioxene, and 20 ml of isopropanol were added. The mixture was heated under reflux for 17 hours. After cooling, the precipitated crystal was collected by filtration and was recrystallized from isopropanol. Thus, 7.89 g (43.2% yield) of the desired product in the form of white crystals was obtained. The analytical data of the product were as follows:

m.p.: 153.5—154.5°C

$IR\nu_{max}^{KBr}cm^{-1}$: 3320 (NH), 1695 (C=O), 1270 (C—O), 1090 (C—O)

NMR ($CDCl_3$, TMS)δ: 1.26 (12H, d, 2 × CH($CH_3$)$_2$), 2.27 (6H, s, 2,6—$CH_3$), 3.90 (4H, s, O—$CH_2$—$CH_2$—O), 4.41 (1H, s, 4—H), 5.04 (2H, m, 2 × $CH$($CH_3$)$_2$), 5.83 (1H, s, vinyl-H), 6.06 (1H, br, NH)

Elementary analysis data (for $C_{19}H_{27}NO_6$)

Calculated: C, 62.45; H, 7.45; N, 3.83(%)

Found: C, 62.66; H, 7.40; N, 3.64(%)

Example 8 (Synthesis Example)

Synthesis of diallyl 4-[2-(5,6-dihydro-p-dioxinyl)]-2,6-dimethyl-1,4-dihydropyridine-3,5-dicarboxylate

A 8.00 g (0.070 mol) amount of 2-formyl-p-dioxene, 21.0 g (0.148 mol) of allyl acetoacetate, and 11.5 ml of a 28% aqueous ammonia were dissolved in 60 ml of isopropanol and the solution was heated under reflux for 24 hours. After cooling, the reaction solution was concentrated until dryness. The resultant residue was treated with ether to crystallize the product. The crystal was collected by filtration and was

recrystallized from isopropanol. As a result, 7.63 g (30.1% yield) of the desired product in the form of pale yellow crystals was obtained.

The analytical data of the product were as follows:

m.p.: 95.5—96.5°C

$IR\nu_{max}^{KBr}cm^{-1}$: 3310 (NH), 1695 (C=O), 1260 (C—O), 1110 (C—O)

NMR ($CDCl_3$, TMS)δ: 2.30 (6H, s, 2,6—$CH_3$), 3.93 (4H, s, O—$CH_2$—$CH_2$—O), 4.53 (1H, s, 4—H), 4.56—4.76 (4H, m, 2 × COO$CH_2$—CH=), 5.05—5.50 (4H, m, 2 × CH=$CH_2$), 5.67—6.30 (2H, m, 2 × CH=$CH_2$), 5.87 (1H, s, dioxene vinyl-H), 6.05 (1H, br, NH)

Elementary analysis data (for $C_{19}H_{23}NO_6$)

Calculated: C, 63.15; H, 6.41; N, 3.88(%)

Found: C, 63.09; H, 6.41; N, 3.77(%)

Example 9 (Synthesis Example)

Synthesis of dipropargyl 4-[2-(5,6-dihydro-p-dioxinyl)]-2,6-dimethyl-1,4-dihydropyridine-3,5-dicarboxylate

A 7.00 g (0.061 mol) amount of 2-formyl-p-dioxene, 18.5 g (0.132 mol) of propargyl acetoacetate, and 10 ml of a 28% aqueous ammonia were dissolved in 60 ml of isopropanol. The mixture was heated under reflux for 24 hours. After cooling, the reaction was concentrated and the precipitated crystal was collected by filtration. The crystal thus obtained was recrystallized from isopropanol to give 7.58 g (34.6% yield) of the desired product in the form of a pale yellow crystal.

The analytical data of the product were as follows:

m.p.: 177.0—179.0°C

$IR\nu_{max}^{KBr}cm^{-1}$: 3340 (NH), 1700 (C=O), 1270 (C—O), 1085 (C—O)

NMR ($CDCl_3$ + $CD_3COCD_3$, TMS)δ: 2.30 (6H, s, 2,6—$CH_3$), 2.56—2.80 (2H, m, 2 × C CH), 3.90 (4H, s, O—$CH_2$—$CH_2$—O), 4.43 (1H, s, 4—H), 4.63—4.80 (4H, m, 2 × COO$CH_2$C≡CH), 5.86 (1H, s, vinyl-H), 7.66 (1H, br, NH)

Elementary analysis data (for $C_{19}H_{19}NO_6$)

Calculated: C, 63.85; H, 5.36; N, 3.92(%)

Found: C, 63.59; H, 5.52; N, 3.73(%)

Example 10 (Synthesis Example)

Synthesis of bis(2-methoxyethyl) 4-[2-(5,6-dihydro-p-dioxinyl)]-2,6-dimethyl-1,4-dihydropyridine-3,5-dicarboxylate

4.56 g (0.04 mol) of 2-formyl-p-dioxene, 12.80 g (0.08 mol) of 2-methoxyethyl acetoacetate, and 4.0 ml of 28% aqueous ammonia were dissolved in 40 ml of isopropanol and the mixture was heated under reflux for 40 hours.

After cooling, the reaction mixture was concentrated until dryness and the residue was dissolved in ethyl acetate. The solution was washed with a small amount of water to remove impurities therefrom. The ethyl acetate phase was dried over anhydrous sodium sulfate and the solvent was then evaporated to give the crystal. The crystal thus obtained was washed with a small amount of ether. The crystal was recrystallized from a mixed solvent of ethyl acetate and n-hexane to give 5.02 g (31.6% yield) of the desired product in the form of white crystals.

The analytical data of the product were as follows:

m.p.: 100.0—101.5°C

$IR\nu_{max}^{KBr}cm^{-1}$: 3280 (NH), 1680 (C=O), 1260 (C—O), 1100 (C—O)

NMR ($CDCl_3$, TMS)δ: 2.30 (6H, s, 2,6—$CH_3$), 3.42 (6H, s, 2 × $OCH_3$), 3.67 (4H, t, 2 × $COOCH_2$—$CH_2$—O), 3.93 (4H, s, O—$CH_2$—$CH_2$—O), 4.30 (4H, m, 2 × COO$CH_2$—$CH_2$—O), 4.50 (1H, s, 4—H), 5.95 (1H, s, vinyl-H), 6.13 (1H, br, NH)

Elementary analysis data (for $C_{19}H_{27}NO_8$)

Calculated: C, 57.42; H, 6.85; N, 3.52(%)

Found: C, 57.58; H, 6.89; N, 3.42(%)

Example 11 (Synthesis Example)

Synthesis of isopropyl methyl 4-[2-(5,6-dihydro-p-dioximyl)]-2,6-dimethyl-1,4-dihydropyridine-3,5-dicarboxylate

a) A 8.64 g (0.06 mol) amount of isopropyl acetoacetate and 6.84 g (0.06 mol) of 2-formyl-p-dioxene were dissolved in 30 ml of isopropanol and 5 drops of piperidine was added thereto. The mixture was allowed to stand for one week at a room temperature while stirring. Then, the solvent was removed by evaporation and the residue was subjected to chromatographic purification by using a column packed with 450 g of silica gel. A mixed solvent of n-hexane and ethyl acetate (1:1) (V/V) was used as an eluent. As a result, 5.89 g (40.8% yield) of isopropyl 1-[2-(5,6-dihydro-p-dioxinyl)]-3-oxo-1-butene-2-carboxylate was obtained.

b) A 3.79 g (0.016 mol) amount of isopropyl 1-[2-(5,6-dihydro-p-dioxinyl)]-3-oxo-1-butene-2-carboxylate and 1.82 g (0.016 mol) of methyl β-aminocrotonate were dissolved in 10 ml of isopropanol and the mixture was heated under reflux for 5 hours. After cooling, the solvent was removed by evaporation. The oil residue thus obtained was crystallized from a mixed solvent of diethyl ether and n-hexane. The

crystal was collected by filtration and was recrystallized from diisopropyl ether. Thus, 1.14 g (21.1% yield) of the desired product in the form of a white crystal was obtained.

The analytical data of the product were as follows:

m.p.: 135.5—137.5°C

$IR\nu_{max}^{KBr}cm^{-1}$: 3320 (NH), 1700 (C=O), 1275 (C—O), 1105 (C—O)

NMR ($CDCl_3$, TMS)δ: 1.27 (6H, d, $CH(CH_3)_2$), 2.29 (6H, s, 2,6—$CH_3$), 3.71 (3H, s, O—$CH_3$), 3.91 (4H, s, O—$CH_2$—$CH_2$—O), 4.43 (1H, s, 4—H), 5.06 (1H, m, $CH(CH_3)_2$), 5.83 (1H, s, vinyl-H), 6.13 (1H, br, NH)

Elementary analysis data (for $C_{17}H_{23}NO_6$)

Calculated: C, 60.52; H, 6.87; N, 4.15(%)
Found: C, 60.34; H, 6.74; N, 4.00(%)

Example 12 (Synthesis Example)

Synthesis of 3,5-diacetyl-4-[2-(5,6-dihydro-p-dioxinyl)]-2,6-dimethyl-1,4-dihydropyridine

A 2.85 g (0.025 mol) amount of 2-formyl-p-dioxene, 2.50 g (0.025 mol) of acetyl acetone, and 2.48 g (0.025 mol) of 4-amino-3-pentene-2-one were mixed and heated at a temperature of 110°C for 24 hours while stirring. After cooling, the reaction mixture was subjected to chromatographic purification by using a column packed with 15 g of silica gel. A mixed solvent of chloroform and ethyl acetate (5:1) (V/V) was used as an eluent. The crystal thus obtained was recrystallized from isopropanol to give 0.97 g (14.0% yield) of the desired product in the form of a pale yellow crystal.

The analytical data of the product were as follows:

m.p.: 186.0—187.0°C

$IR\nu_{max}^{KBr}cm^{-1}$: 3260 (NH), 1660 (C=O), 1230 (C—O)

NMR ($CDCl_3$, TMS)δ: 2.30 (6H, s, 2,6—$CH_3$), 2.34 (6H, s, 2 × $COCH_3$), 3.93 (4H, s, —O$CH_2$—$CH_2$—O), 4.50 (1H, s, 4—H), 5.75 (1H, s, vinyl-H), 7.35 (1H, br, NH)

Elementary analysis data (for $C_{15}H_{19}NO_4$)

Calculated: C, 64.97; H, 6.91; N, 5.05(%)
Found: C, 65.09; H, 6.80; N, 4.90(%)

Example 13 (Synthesis Example)

Synthesis of 3,5-diacetyl-4-[2-(5,6-dihydro-p-dioxinyl)]-2,6-dimethyl-1,4-dihydropyridine

A 20.0 g (0.175 mol) amount of 2-formyl-p-dioxene, 18.5 g (0.185 mol) of acetyl acetone, and 18.1 g (0.183 mol) of 4-amino-3-penten-2-one were dissolved in 100 ml of pyridine and the mixture was heated under reflux for 48 hours. After cooling, the reaction mixture was concentrated until dryness and the residue was crystallized by treating it with diethyl ether. The crystal was collected by filtration and was then recrystallized from isopropanol. Thus, 18.0 g (37.0% yield) of the desired product in the form of a pale yellow crystal was obtained.

Example 14 (Synthesis Example)

Synthesis of methyl 5-acetyl-4-[2-(5,6-dihydro-p-dioxinyl)]-2,6-dimethyl-1,4-dihydropyridine-3-carboxylate

a) A 11.6 g (0.10 mol) amount of methyl acetoacetate and 11.4 g (0.10 mol) of 2-formyl-p-dioxene were dissolved in 50 ml of isopropanol and 10 drops of piperidine were added thereto. The mixture was allowed to stand for 5 days at a temperature of 40°C to 60°C while stirring. Then, the solvent was removed from the reaction mixture by evaporation. The residue was subjected to chromatographic purification by using a column packed with 680 g of silica gel. A mixed solvent of benzene and ethyl acetate (3:1) (V/V) was used as an eluent. Thus, 4.02 g (19.0% yield) of oily methyl 1-[2-(5,6-dihydro-p-dioxinyl)]-3-oxo-1-butene-2-carboxylate was obtained.

b) A 4.02 (0.019 mol) amount of methyl 1-[2-(5,6-dihydro-p-dioxinyl)]-3-oxo-1-butene-2-carboxylate and 1.88 g (0.019 mol) of 4-amino-3-penten-2-one were dissolved in 15 ml of isopropanol and the mixture was heated under reflux for 10 hours. After cooling, the solvent was removed by evaporation and the oily residue was crystallized by treating it with diethyl ether. The crystal thus obtained was collected by filtration. The crystal was recrystallized from ethanol to give 2.16 g (38.8% yield) of the desired product in the form of a pale yellow crystal.

The analytical data of the product were as follows:

m.p.: 159.5—160.5°C

$IR\nu_{max}^{KBr}cm^{-1}$: 3320 (NH), 1710 (C=O), 1670 (C=O), 1220 (C—O), 1120 (C—O)

NMR ($CDCl_3$, TMS)δ: 2.27 (3H, s, $COCH_3$), 2.30 (6H, s, 2,6—$CH_3$), 3.73 (3H, s, O—$CH_3$), 3.91 (4H, s, O—$CH_2$—$CH_2$—O), 4.46 (1H, s, 4—H), 5.78 (1H, s, vinyl-H), 6.47 (1H, br, NH)

Elementary analysis data (for $C_{15}H_{19}NO_5$)

Calculated: C, 61.42; H, 6.53; N, 4.78(%)
Found: C, 61.24; H, 6.48; N, 4.66(%)

Example 15 (Synthesis Example)

Synthesis of 2-methoxyethyl 5-acetyl-4-[2-(5,6-dihydro-p-dioxinyl)]-2,6-dimethyl-1,4-dihydropyridine-3-carboxylate

A 2.96 g (0.03 mol) amount of 4-amino-3-penten-2-one, 4.79 g (0.03 mol) of 2-methoxyethyl

acetoacetate, and 3.39 g (0.03 mol) of 2-formyl-p-dioxene were dissolved in 15 ml of isopropanol and the mixture was heated under reflux for 16 hours. After cooling, the reaction mixture was concentrated until dryness and the residue was subjected to chromatograph purification by using a column packed with 500 g of silica gel. A mixed solvent of benzene and acetone (3:1) (V/V) was used as an eluent. The purified product was crystallized from diethyl ether. The crystal was recrystallized from a mixed solvent of diisopropyl ether and diethyl ether and was further recrystallized from diethyl ether. Thus, 0.55 g of the desired pale yellow product was obtained.

The analytical data of the product were as follows:

m.p.: 106.0—110.0°C

IR$\nu_{max}^{KBr}$cm$^{-1}$: 3310 (NH), 1700 (C=O), 1670 (C=O), 1220 (C—O)

NMR (CDCl$_3$, TMS)δ: 2.30 (9H, s, 2,6—CH$_3$, CO—CH$_3$), 3.40 (3H, s, O—CH$_3$), 3.63 (2H, t, COO—CH$_2$—*CH$_2$*—O), 3.93 (4H, s, O—*CH$_2$*—*CH$_2$*—O), 4.33 (2H, m, COO—*CH$_2$*—CH$_2$—O), 4.47 (1H, s, 4—H), 5.85 (1H, s, vinyl-H), 6.15 (1H, br, NH)

Elementary analysis data (for $C_{17}H_{23}NO_6$)

| | | | |
|---|---|---|---|
| Calculated: | C, 60.52; | H, 6.87; | N, 4.15(%) |
| Found: | C, 60.30; | H, 6.77; | N, 3.98(%) |

Example 16 (Evaluation Example)

Effect of the compounds according to the present invention on liver disorder induced by carbon tetrachloride.

The effects of the sample compounds to be tested on hepatic disorder were evaluated by determining the levels of GOT, GPT, AlP, and bilirubin, which are known as an indicator of hepatic disorder, in serum, after administering carbon tetrachloride and the sample compounds to SLC-Wistar male rats (9 weeks old and body weight of 210 to 250 g in Tables 1, 3 and 4, and 10 weeks old and body weight of 230 to 260 g in Table 2) as follows.

Five rats were used in each test group. The sample compound (i.e., each compound listed below) was suspended at a concentration of 40 mg/ml in a 0.1% aqueous solution of Tween-80 (i.e., nonionic surfactant available from Atlas Powder Co., Ltd.). The resultant suspension was orally administered at a dose of 200 mg/kg each at 25 hours and 1 hour before the administration of carbon tetrachloride, except for the control group. For the control group to which only carbon tetrachloride was administered in the evaluation test, a 0.1% aqueous Tween-80 was orally administered at a dose of 5 ml/kg in the same manner.

Carbon tetrachloride was intraperiotoneally administered as a 10% olive oil solution at a dose of 5 ml/kg (0.5 ml/kg in terms of CCl$_4$) one hour after the final administration of the sample compound. For the control (normal) group, olive oil was intraperitoneally administered in the same manner at a dose of 5 ml/kg.

Blood samples were collected from the carotid artery under pentobarbital anesthesia, 24 hours after the administration of carbon tetrachloride. The rats were fasted for 18 to 22 hours before collecting the blood samples.

The determination of GOT and GPT in serum was carried out according to a UV method, the determination of AlP was carried out according to Bessy-Lowry method, and the determination of bilirubin was carried out according to a diazo method. The results are shown in Tables 1 to 4.

The sample compounds A to Q are as follows:

| Example No. | Sample Compound |
|---|---|
| 1 | A |
| 2 | B |
| 3 | C |
| 4 | D |
| 6 | F |
| 7 | G |
| 8 | H |
| 9 | I |
| 10 | J |
| 11 | K |
| 12 | L |
| 14 | M |
| 15 | N |

TABLE 1

| | GOT (K.U.) | GPT (K.U.) | AlP (K—AU.) | Bilirubin (mg/dl) |
|---|---|---|---|---|
| Control (normal) | 76 ± 1 | 31 ± 2 | 23.6 ± 0.4 | 0.10 ± 0.00 |
| $CCl_4$ | 17327 ± 2336 | 10496 ± 1221 | 35.5 ± 2.5 | 0.62 ± 0.30 |
| A + $CCl_4$ | 3874 ± 792 | 2380 ± 472 | 31.4 ± 0.9 | 0.24 ± 0.02 |
| B + $CCl_4$ | 1604 ± 569 | 1023 ± 368 | 34.1 ± 3.4 | 0.20 ± 0.00 |
| D + $CCl_4$ | 2572 ± 518 | 2050 ± 419 | 31.0 ± 1.5 | 0.20 ± 0.00 |

TABLE 2

| | GOT (K.U.) | GPT (K.U.) | AlP (K—AU.) | Bilirubin (mg/dl) |
|---|---|---|---|---|
| Control (normal) | 89 ± 12 | 41 ± 3 | 22.7 ± 0.6 | 0.10 ± 0.00 |
| $CCl_4$ | 20701 ± 2821 | 11034 ± 1354 | 32.3 ± 2.8 | 0.40 ± 0.09 |
| C + $CCl_4$ | 1447 ± 313 | 912 ± 198 | 31.7 ± 3.2 | 0.20 ± 0.00 |

TABLE 3

| | GOT (K.U.) | GPT (K.U.) | AlP (K—AU.) | Bilirubin (mg/dl) |
|---|---|---|---|---|
| Control (normal) | 81 ± 3 | 30 ± 2 | 27.0 ± 0.5 | 0.22 ± 0.04 |
| $CCl_4$ | 13189 ± 2205 | 9188 ± 1815 | 34.4 ± 2.6 | 0.38 ± 0.06 |
| F + $CCl_4$ | 432 ± 107 | 274 ± 99 | 26.4 ± 1.0 | 0.22 ± 0.05 |

TABLE 4

| | GOT (K.U.) | GPT (K.U.) | AIP (K—AU.) | Bilirubin (mg/dl) |
|---|---|---|---|---|
| Control (normal) | 76 ± 4 | 31 ± 3 | 24.4 ± 0.7 | 0.10 ± 0.00 |
| $CCl_4$ | 17704 ± 4140 | 10523 ± 2313 | 35.0 ± 2.0 | 0.38 ± 0.04 |
| G + $CCl_4$ | 3482 ± 707 | 2994 ± 638 | 31.5 ± 1.2 | 0.22 ± 0.02 |
| H + $CCl_4$ | 1920 ± 881 | 1453 ± 699 | 29.5 ± 2.4 | 0.22 ± 0.04 |
| I + $CCl_4$ | 2925 ± 380 | 2110 ± 228 | 27.6 ± 1.8 | 0.22 ± 0.02 |
| J + $CCl_4$ | 807 ± 211 | 626 ± 179 | 27.4 ± 2.6 | 0.18 ± 0.03 |
| K + $CCl_4$ | 2290 ± 619 | 1657 ± 436 | 29.6 ± 0.8 | 0.24 ± 0.02 |
| L + $CCl_4$ | 429 ± 113 | 285 ± 96 | 34.2 ± 1.5 | 0.22 ± 0.02 |
| M + $CCl_4$ | 807 ± 176 | 428 ± 88 | 29.1 ± 1.1 | 0.20 ± 0.00 |
| N + $CCl_4$ | 3430 ± 922 | 2860 ± 858 | 28.5 ± 1.7 | 0.18 ± 0.02 |

Example 17 (Evaluation Example)

Acute toxicity of the compounds according to the present invention.

$LD_{50}$ values of the present compounds H, I, J, and L used in Example 16 were determined by observing SLC-Wistar male rats (5 weeks old and a body weight of 130 to 150 g) for one week after orally administering the sample compounds to be tested. The results were as follows:

| Compounds | $LD_{50}$ |
|---|---|
| H | more than 1000 mg/kg |
| I | more than 2000 mg/kg |
| J | more than 1000 mg/kg |
| L | more than 1000 mg/kg |

Example 18 (Evaluation Example)

Acute toxicity of the compounds according to the present invention.

$LD_{50}$ values of the present compounds A and F used in Examples 16 were determined by observing SLC-Wistar male rats (5 weeks old and a body weight of 100 to 125 g) for one week after orally administering the sample compounds to be tested. The results were as follows:

| Compounds | $LD_{50}$ |
|---|---|
| A | more than 1000 mg/kg |
| F | more than 2000 mg/kg |

As is clear from the results shown in the above-mentioned Examples, the present 1,4-dihydropyridine compounds are effective for the treatment and prevention of hepatic disorders and also have a low toxicity. Accordingly, the present compounds are suitable for use as a medicine for treatment of hepatic disease caused by various factors.

**Claims**

1. 1,4-Dihydropyridine compound having the general formula:

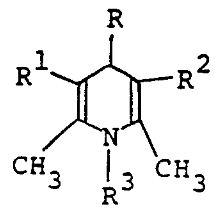

(I)

wherein R is 2-(5,6-dihydro-p-dioxinyl); $R^1$ and $R^2$ are independently a cyano group, an acetyl group, or a substituted oxycarbonyl group having the general formula $COOR^4$ wherein $R^4$ is a methyl, isopropyl, allyl propargyl, methoxymethyl, 2-methoxyethyl, or 1-methyl-2-methoxyethyl group provided that both $R^1$ and $R^2$ are not $—COOCH_3$; and $R^3$ is hydrogen.

2. 1,4-Dihydropyridine compound as claimed in claim 1, wherein $R^1$ and $R^2$ are independently an acetyl group or a substituted oxycarbonyl group having the general formula $COOR^4$ wherein $R^4$ is a methyl, methoxymethyl, or 2-methoxyethyl group provided that both $R^1$ and $R^2$ are not $—COOCH_3$ in the general formula (I).

3. 1,4-Dihydropyridine compound as claimed in claim 2, wherein $R^1$ and $R^2$ in the general formula (I) are the same groups.

4. 1,4-Dihydropyridine compound as claimed in claim 1, wherein $R^1$ and $R^2$ in the general formula (I) are the same groups.

**Patentansprüche**

1. 1,4-Dihydropyridin-Verbindung mit der allgemeinen Formel:

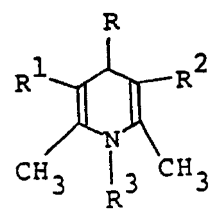

worin R 2-(5,6-Dihydro-p-dioxinyl)darstellt, $R^1$ und $R^2$ unabhängig voneinander eine Cyanogruppe, eine Acetylgruppe oder eine substituierte Oxycarbonylgruppe mit der allgemeinen Formel $COOR^4$ sind, worin $R^4$ eine Methylgruppe, eine Isopropylgruppe, eine Allylgruppe, eine Propargylgruppe, eine Methoxymethylgruppe, eine 2-Methoxyethylgruppe ode eine 1-Methyl-2-methoxyethylgruppe ist, vorausgesetzt, daß sowohl $R^1$ als auch $R^2$ nicht $—COOCH_3$ sind, und $R^3$ Wasserstoff ist.

2. 1,4-Dihydropyridin-Verbindung nach Anspruch 1 worin $R^1$ und $R^2$ unabhängig voneinander eine Acetylgruppe oder eine substituierte Oxycarbonylgruppe mit de allgemeinen Formel $COOR^4$ darstellen, worin $R^4$ eine Methylgruppe, eine Methoxymethylgruppe oder eine 2-Methoxyethylgruppe ist, vorausgesetzt, daß sowohl $R^1$ und $R^2$ in der allgemeinen Formel (I) nicht $—COOCH_3$ darstellen.

3. 1,4-Dihydropyridin-Verbindung nach Anspruch 2, worin $R^1$ und $R^2$ in der allgemeinen Formel (I) die gleichen Gruppen darstellen.

4. 1,4-Dihydropyridin-Verbindung nach Anspruch 1, worin $R^1$ und $R^2$ in der allgemeinen Formel (I) die gleichen Gruppen darstellen.

**Revendications**

1. 1,4-dihydropyridines présentant la formule générale:

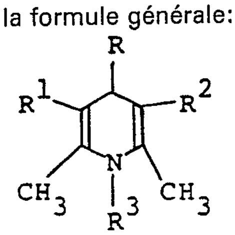

(I)

dans laquelle:

R est 2-(5,6-dihydro-p-dioxinyl);

$R^1$ et $R^2$, indépendamment l'un de l'autre, consistent en un groupe cyano, un groupe acétyle ou un groupe oxycarbonyle substitué, présentant la formule générale $COOR^4$, dans laquelle $R^4$ est un radical

méthyle, isopropyle, allyle, propargyle, méthoxyméthyle, 2-méthoxyéthyle ou 1-méthyl-2-méthoxyéthyle étant entendu que $R^1$ et $R^2$ ne peuvent pas être simultanément un radical $-COOCH_3$; et

$R^3$ est atome d'hydrogène.

2. 1,4-dihydropyridines ainsi que revendiquées dans la revendication 1, dans la formule générale I dans laquelle $R^1$ et $R^2$, indépendamment l'un de l'autre, consistent en un groupe acétyle ou en un groupe oxycarbonyle substitué présentant la formule $COOR^4$ dans laquelle $R^4$ est un radical méthyle, méthoxyméthyle ou 2-méthoxyéthyle, étant entendu que $R^1$ et $R^2$ ne peuvent pas être simultanément un radical $COOCH_3$.

3. 1,4-dihydropyridines ainsi que revendiquées dans la revendication 2, dans la formule générale I dans laquelle $R^1$ et $R^2$ sont des groupes identiques.

4. 1,4-dihydropyridines ainsi que revendiquées dans la revendication 1, dans la formule générale I dans laquelle $R^1$ et $R^2$ sont identiques.